**Europäisches Patentamt**

**European Patent Office**

**Office européen des brevets**

(19)

(11) Veröffentlichungsnummer: **0 140 110**
**B1**

(12)

# EUROPÄISCHE PATENTSCHRIFT

(45) Veröffentlichungstag der Patentschrift:
02.09.87

(21) Anmeldenummer: 84110999.4

(22) Anmeldetag: 14.09.84

(51) Int. Cl.⁴: **A 23 J 3/00,** A 23 L 1/30, A 23 K 1/04 // A61K35/14, A61K7/00, A01N1/02

(54) **Verfahren zur Verarbeitung von Tiervollblut.**

(30) Priorität: 28.10.83 PL 244394

(43) Veröffentlichungstag der Anmeldung:
08.05.85 Patentblatt 85/19

(45) Bekanntmachung des Hinweises auf die Patenterteilung:
02.09.87 Patentblatt 87/36

(84) Benannte Vertragsstaaten:
BE CH DE FR GB IT LI NL SE

(56) Entgegenhaltungen:
WO-A-84/00098
DE-C-114 823
FR-A-2 467 550

CHEMICAL ABSTRACTS, Band 89, Nr. 15, Oktober
1978, Seite 462, Nr. 127880e, Columbus, Ohio, US;
A.I. ZHARINOV et al.: "Use of precipitated blood
plasma proteins in the production of canned meat
paste products"
CHEMICAL ABSTRACTS, Band 99, Nr. 1, Juli 1983,
Seite 413, Nr. 4397b, Columbus, Ohio

Die Akte enthält technische Angaben, die nach
dem Eingang der Anmeldung eingereicht wurden
und die nicht in dieser Patentschrift enthalten sind.

(73) Patentinhaber: **Akademia Rolnicza, ul. Norwida 25,
Wroclaw (PL)**

(72) Erfinder: **Zaleski, Stanislaw, Wroclaw ul, Canaletta
16/5 (PL)**
Erfinder: **Tereszkiewicz, Ryszard, Wroclaw ul,
Wróblewskiego 25 (PL)**
Erfinder: **Lawik, Bogumil, Wroclaw ul, Canaletta
2a/5 (PL)**
Erfinder: **Kierzkowski, Marian, Wroclaw ul,
Zachodnia 4/8 (PL)**

(74) Vertreter: **Hansen, Bernd, Dr.rer.nat., Hoffmann,
Eitle & Partner Patentanwälte Arabellastrasse 4,
D-8000 München 81 (DE)**

**Beschreibung**

Die Erfindung betrifft ein Verfahren zur Verarbeitung von Tiervollblut, das für Verbrauchs- und Futterzwecke, pharmazeutische Zwecke oder Schönheitszwecke bestimmt ist.

Die Blutstabilisierung zur Reaktion auf den Erstarrungsprozess und die Blutaufbewahrung im flüssigen Zustand wird unter anderem mit Hilfe von Salzen anorganischer Säuren, überwiegend Kochsalz, durchgeführt. Letzteres wird z. B. dem Schweineblut in einer Menge von mindestens 3 Massenprozent zugegeben. Aus praktischen Gründen wird der Stabilisator in grösserer Menge, d.h. bis zu 20 Massenprozent, eingeführt. Das stabilisierte Tierblut, vor allem das Schweineblut, wird im allgemeinen zu Verbrauchszwecken ausgenutzt, z. B. zur Herstellung von Blutwürsten oder Grützwürsten, wobei seine Menge entsprechend der Bindungskraft oder der Wasseraufnahmefähigkeit der restlichen Komponenten beschränkt ist und praktisch 30 Massenprozent des Produktes nicht überschreitet.

Aus PL-PS 122 519 ist ein Verfahren zur Härtung von Tierblut für Verbrauchs- oder Futterzwecke bekannt, welches in einem Vermischen von 5 bis 60 Massenprozent Käsewasser oder Magermilch, 5 bis 10 Massenprozent Ferment von Gärungsmilchbakterien und 30 bis 90 Massenprozent Tierblut besteht. Daraufhin wird dieses Gemisch der Temperatureinwirkung von 288 bis 313° K bis zum Erhalt von mit Gel ausgefüllten Gerinnseln und dem Brühen in Wasser bei einer Temperatur von 343 bis 383° K, bis die ganze Masse eine schokoladenbraune Farbe angenommen hat, unterzogen.

Aus der polnischen Patentanmeldung P 238 875 ist ein Verfahren zur Darstellung von Gelgerinnseln für Verbrauchs- oder Futterzwecke unter Verwendung von stabilisiertem Blut und Käsewasser, Sauermilch oder deren Gemisch bekannt, das in einer Ergänzung dieses Gemisches durch Tiergalle in einer Menge von 0,1 bis 5,0 Massenprozent und hefeabgeleiteten Substanzen in einer Menge von 1,0 bis 15,0 Massenprozent, sowie Verbrauchszubereitungen zur Verbesserung des Geschmacks besteht. Das Gelgerinnsel kann in Formverpackungen dargestellt sein. Das auf diese Weise dargestellte frische Gelgerinnsel eignet sich zum Verzehr direkt nach dem Braten in einem Ölbad mit den Geschmacksmitteln. Es schmeckt fast wie Leber.

Die beschriebenen Verfahren sind dadurch charakterisiert, dass man in der Praxis nur von einem Teil des Gemisches die Produktdarstellung durchführt, was ökonomische Verluste und einen Nährstoffverlust des Produkts zur Folge hat.

Dieser Effekt ist ähnlich dem bekannten Retraktionseffekt, der während des Erstarrens des soeben aus den Gefässen entfernten Blutes auftritt, d.h. dass in dem dreidimensionalen Fibrinnetz, in dessen Maschen die flüssigen und geformten Blutkomponenten enthalten sind, nach einiger Zeit ein Schrumpfen von Kleinfasern und ein Auspressen von Serum erfolgt.

Die Erfindung betrifft ein Verfahren zur Verarbeitung von Tiervollblut unter Zugabe von Alkalisalzen und ist dadurch gekennzeichnet, daß man dem frisch aus den Gefäßen entfernten Schlachttierblut Alkalisalze, vorzugsweise Natrium- oder Kaliumchlorid, bis zu einer Elektrolytkonzentration von 0,085 bis 0,42 Mol/dm³ entsprechend einer Konzentration von 0,5 bis 2,5 Massenprozent Natriumchlorid zugibt, das erhaltene Produkt erstarren läßt und dann thermisch härtet.

Beim beanspruchten Verfahren gibt man vorzugsweise eine organische oder anorganische Säure bis zu einem pH-Wert von 5,7 bis 6,5 zu. Weiterhin kann es vorteilhaft sein, zur Verbesserung der Nährwerte oder der Heileigenschaften Vitamine, Aminosäuren und Spurenelemente sowie Geschmacks- und Geruchssubstanzen zuzugeben.

Gemäß einer Ausführungsform wird dem mit Alkalisalzen stabilisierten Blut ein Verdünnungsmittel mit neutraler Reaktion in Form des frisch aus den Gefäßen entfernten Blutes, Wasser und flüssige eiweiß- und/oder kohlenhydrathaltige Substanzen zugegeben.

Gemäß einer weiteren Ausführungsform gibt man dem mit Alkalisalzen stabilisierten Blut das Verdünnungsmittel mit saurer Reaktion in Form von wässrigen Lösungen und/oder eiweiß- oder kohlenhydrathaltigen Substanzen bis zu einem pH von nicht niedriger als 5,7 zu.

Gemäss der Erfindung wurde ganz unerwartet festgestellt, dass durch Auswahl eines bestimmten Konzentrationsbereiches von Alkalimetallen im Blut und durch Einstellung einer bestimmten Kationenkonzentration der typische Prozess der Bluterstarrung modifiziert werden kann.

Falls man dem Blut ein Verdünnungsmittel zugibt, ist im Bereich des aufgequollenen Fibrinnetzes zusätzlich dieses Verdünnungsmittel enthalten.

Sowohl bei Anwendung von reinem Blut als auch in Kombination mit Verdünnungsmitteln, hat das Rohprodukt, das mit dem Verfahren gemäss der Erfindung hergestellt wird, die Form einer harten Gallerte mmit lebendig roter Farbe wobei der Effekt der Retraktion ausbleibt und das Rohprodukt aus dem vollen Volumen aller Komponenten gewonnen wird. Durch thermische Härtung des Rohproduktes erfolgt eine Veränderung der Struktur, wobei die gebildete Konsistenz irreversibel und dem Eiweiss des Hühnereies, das gekocht wurde, ähnlich ist. Das Produkt selbst verändert die Farbe zu braun. In Abhängigkeit von den Komponenten, die zur Herstellung verwendet wurden, gibt es zahlreiche Möglichkeiten das Produkts zu verwenden. Für Verbrauchs- und Futterzwecke ist es bevorzugt, das Rohprodukt dem Pasteurisationsprozess zu unterziehen, der neben einer Verminderung der Bakterienanzahl gleichzeitig zur Produkthärtung führt, wobei sich letzteres in dieser Form entweder zum direkten Verzehr, zum Zerstückeln und zur Zugabe zu anderen Verbrauchs- oder Futterprodukten und zur Herstellung neuer Speisen oder Futtergemische eignet.

Die Futtergemische, die durch Zugabe des erfindungsgemässen Produktes hergestellt wurden, sind charakterisiert durch eine hohe Resorption, leichte Verdaulichkeit und durch Gewinnung eines grösseren Gewichtszuwachses bei einem reduzierten Verbrauch von Futter im Vergleich zu den bekannten Komponenten.

**Beispiel 1**

In eine Vorrichtung mit einem Volumen von 10 l werden 4 g Kochsalz eingeschüttet, worauf 5 l des frisch aus den Gefässen entfernten Rinderblut eingegossen werden. Daraufhin wird die Vorrichtung für ca. 90 Minuten bei Zimmertemperatur stehen gelassen. Das erhaltene Rohprodukt wird in Stücke geteilt, deren Dicke 40 mm nicht überschreitet, und im Wasser bei einer Temperatur von 353° K 40 Minuten lang pasteurisiert. Das Produkt, das auf diese Weise hergestellt wurde, eignet sich bevorzugt zu Futterzwecken.

**Beispiel 2**

In eine Vorrichtung mit einem Volumen von 100 l werden 20 kg Kochsalz eingeschüttet, und bis auf 100 l mit soeben aus den Gefässen entfernten Schweineblut ergänzt. Zunächst werden nach völliger Salzauflösung 7,5 l Blut dem beliebige wasserlösliche Vitamine in einer Menge von 2 g zugegeben worden sind, zugegeben. Dann werden weitere 92,5 l frisch aus den Gefässen entferntes Schweineblut zugegeben. Das Ganze wird 90 Minuten lang stehen gelassen. Das Rohprodukt wird dem nach Zerschneiden in Stücke bei einer Temperatur von 353 bis 363° K während 30 bis 40 Minuten pasteurisiert; es ist für pharmazeutische Zwecke, zur Herstellung eines antianämischen Spezifikums bestimmt.

**Beispiel 3**

In einen Behälter mit einem Volumen von 50 l werden 1,30 kg Kochsalz eingeschüttet, worauf 6 l frisch aus den Gefässen entfernten Schweineblutes in den Behälter eingegossen werden. Das zugegebene Kochsalz wird durch intensives Vermischen mit dem frisch aus den Gefässen entfernten Blut verteilt und dann wird das Blut bis auf ein Volumen von 50 l aufgefüllt. Die auf diese Weise gewonnene Lösung wird mit durch Schwefelsäure angesäuertem Wasser im Verhältnis 50:50 gebunden, unter Einhaltung eines pH-Wertes von ca. 6,0 des Blut- und Wassergemisches. Nach 90 Minuten gewinnt man das Rohprodukt, das nach Zerschneiden in Stücke der Grösse 100 bis 150 g in Wasser bei einer Temperatur von 353 bis 363° K während 30 bis 40 Minuten pasteurisiert wird. Das neugewonnene Produkt kann zur Tierzucht verwendet oder dem Gefrieren und Trocknen zur Verlängerung seiner Dauerhaftigkeit unterzogen werden.

**Beispiel 4**

In Behälter mit einem Volumen von 15 l werden 8 dag Kochsalz eingeschüttet; daraufhin werden 5 l frisch aus den Gefässen entferntes Schweineblutes eingegossen. Nach vollständiger Auflösung des Kochsalz im Blut werden 5 l Fleischbrühe zugegossen. In das gewonnene Gemisch werden 50 g Schweinegalle eingeführt. Das Ganze wird bis zu 90 Minuten stehen gelassen.

Das erhaltene Produkt wird in Stücke von 100 bis 150 g geschnitten und der thermischen Verarbeitung bei einer Temperatur von 403° K während 3 Minuten im Ölbad unterzogen wird. Das auf diese Art gewonnene Produkt ähnelt täuschend der Tierleber.

**Beispiel 5**

In Behälter mit einem Volumen von 15 l werden 80 g Kochsalz eingeschüttet; daraufhin werden 7 l frisch aus den Gefässen entfernte Schweineblute eingegossen, und nach Salzauflösung werden 3 l Mohrrübensaft zugegossen. Das Ganze wird mit Zitronensäure derart angesäuert, dass der pH-Wert des Gemisches ca. 6,0 beträgt. Das Ganze wird 90 Minuten lang stehen gelassen. Das Rohprodukt wird nach dem Zerschneiden in Stücke von 100 bis 150 bei einer Temperatur von 353 bis 363° K während 30 bis 40 Minuten pasteurisiert, wodurch man direkt das Produkt mit der gewünschten kulinarischen Qualität gewinnt.

**Patentansprüche**

1. Verfahren zur Verarbeitung von Tiervollblut unter Zugabe von Alkalisalzen, dadurch gekennzeichnet, daß man dem frisch aus den Gefäßen entfernten Schlachttierblut Alkalisalze, vorzugsweise Natrium- oder Kaliumchlorid, bis zu einer Elektrolytkonzentration von 0,085 bis 0,43 Mol/dm³ entsprechend einer Konzentration von 0,5 bis 2,5 Massenprozent Natriumchlorid zugibt, das erhaltene Produkt erstarren läßt und dann thermisch härtet.

2. Verfahren nach Anspruch 1, dadurch gekennzeichnet, daß man eine organische oder anorganische Säure bis zu einem pH-Wert von 5,7 bis 6,5 zugibt.

3. Verfahren nach Anspruch 1, dadurch gekennzeichnet, daß man Vitamine, Aminosäuren und Spurenelemente zugibt.

4. Verfahren nach Anspruch 1, dadurch gekennzeichnet, daß man Geschmacks- und Geruchssubstanzen zugibt.

5. Verfahren zur Verarbeitung von Tiervollblut gemäß Ansprüchen 1 bis 4, dadurch gekennzeichnet, daß man dem mit Alkalisalzen stabilisierten Blut Verdünnungsmittel mit neutraler Reaktion in Form des frisch aus den Gefäßen entfernten Blutes, Wasser und flüssiger eiweiß- und/oder kohlenhydrathaltiger Substanzen zugibt.

6. Verfahren zur Verarbeitung von Tiervollblut gemäß Ansprüchen 1 bis 4, dadurch gekennzeichnet, daß man dem mit Alkalisalzen stabilisierten Blut das Verdünnungsmittel mit saurer Reaktion in Form von wässrigen Lösungen und/oder eiweiß- oder kohlenhydrathaltigen Substanzen bis zu einem pH-Wert von nicht niedriger als 5,7 zugibt.

## Claims

1. Process for working up full animal blood with the addition of alkali salts, characterized in that alkali salts, preferably sodium chloride or potassium chloride, are added to blood freshly removed from the vessels of animals for slaughter up to an electrolyte concentration of 0.085 to 0.43 mol/dm³ corresponding to a concentration of 0.5 to 2.5 mass % of sodium chloride, the product obtained is allowed to congeal, and then thermally hardened.

2. Process according to claim 1, characterized in that an organic or inorganic acid is added up to a pH value of 5.7 to 6.5.

3. Process according to claim 1, characterized in that vitamins, amino acids, and trace elements are added.

4. Process according to claim 1, characterized in that flavoring and aromatic substances are added.

5. Process for working up full animal blood according to any of claims 1 to 4, characterized in that diluents having a neutral reaction in the form of blood freshly removed from the vessels, water and liquid protein and/or carbohydrate-containing substances are added to the blood stabilized with alkali salts.

6. Process for working up full animal blood according to any of claims 1 to 4, characterized in that diluents with an acid reaction in the form of aqueous solutions and/or protein or carbohydrate-containing substances are added to the blood stabilized with alkaline salts up to a pH value of not lower than 5.7.

## Revendications

1. Procédé pour traiter le sang animal entier par addition de sels alcalins, caractérisé en ce que l'on ajoute au sang d'animal d'abattage fraîchement prélevé à partir des vaisseaux, des sels alcalins, de préférence du chlorure de sodium ou de potassium, jusqu'à une concentration en électrolyte de 0,085 à 0,43 mole/l, ce qui correspond à une concentration de 0,5 à 2,5 % en poids de chlorure de sodium, on laisse solidifier le produit puis on le durcit à la chaleur.

2. Procédé selon la revendication 1, caractérisé en ce que l'on ajoute un acide organique ou minéral jusqu'à pH 5,7-6,5.

3. Procédé selon la revendication 1, caractérisé en ce que l'on ajoute des vitamines, des aminoacides et des oligoéléments.

4. Procédé selon la revendication 1, caractérisé en ce que l'on ajoute des substances aromatisantes.

5. Procédé pour traiter du sang animal entier selon les revendications 1 à 4, caractérisé en ce que l'on ajoute au sang stabilisé par les sels alcalins des diluants à réaction neutre sous forme de sang fraîchement prélevé sur des animaux, d'eau et de substances liquides contenant des protéines et/ou des hydrates de carbone.

6. Procédé pour traiter du sang animal entier selon les revendications 1 à 4, caractérisé en ce que l'on ajoute au sang stabilisé par des sels alcalins, le diluant à réaction acide sous forme d'une solution aqueuse et/ou de substances contenant des protéines ou des hydrates de carbone jusqu'à un pH non inférieur à 5,7.